# EUROPEAN PATENT SPECIFICATION

(11) **EP 2 010 087 B1**
(45) Date of publication and mention of the grant of the patent: **05.11.2014**
(21) Application number: 07760347.0
(22) Date of filing: 09.04.2007
(51) Int. Cl.: A61B 18/20

(54) **APPARATUS FOR PRODUCING THERMAL DAMAGE WITHIN THE SKIN**
VORRICHTUNG ZUR ERZEUGUNG THERMISCHER SCHÄDEN IN DER HAUT
DISPOSITIF POUR PRODUIRE UNE LÉSION THERMIQUE DANS LA PEAU

(30) Priority: 07.04.2006 US 790172 P
(43) Date of publication of application: 07.01.2009
(60) Divisional application: 12194020.9
(73) Proprietor: THE GENERAL HOSPITAL CORPORATION, Boston, MA 02214 (US)
(72) Inventor: MANSTEIN, Dieter, Boston, MA 02114 (US)
(74) Representative: Kopf, Korbinian Paul
(86) International application number: PCT/US2007/066264
(87) International publication number: WO 2007/118244

(56) References cited:
- EP-A- 1 627 662
- WO-A-91/13652
- US-A- 4 408 602
- US-A- 5 522 813
- US-A- 6 110 195
- US-A1- 2006 149 223

## Description

### FIELD OF THE INVENTION

The present invention relates to an apparatus that uses an electromagnetic radiation for a dermatological treatment and, more particularly, to an apparatus that can use optically-generated radiation to ablate a passage into the skin, with another radiation then being directed into the passage to create localized thermal damage deep within the skin.

### BACKGROUND INFORMATION

There is currently an increased demand for a repair of skin defects, which can be induced by aging, sun exposure, dermatological diseases, traumatic effects, and the like. A number of treatments which utilize an electromagnetic radiation have been used to improve skin defects by inducing a thermal injury to the skin, which generally results in a complex wound healing response of the skin. This can lead to a biological repair of the injured skin. Examples of such treatments can be found in US 6 110 195, WO 91/13652 or US 5522 813.

The electromagnetic radiation can undergo complex reactions with biological tissues, including the skin. The degree of interaction between the biological tissues and the radiation can be affected by characteristics of both the tissues and the radiation. Chromophores within the skin, such as melanin and hemoglobin, can absorb different frequencies of light. Water can act as a chromophore for the radiation provided by certain types of lasers, leading to high absorption rates and little penetration. Radiation energy can also be scatter as it passes through skin and other biological tissues.

The absorption of the electromagnetic radiation passing through the skin can lead to the generation of heat at the absorption site. This effect can be used to generate targeted heating of local regions of the tissue based on the absorption properties. For example, preferential absorption by dark hair can be used in laser hair removal techniques, where the hair follicles are thermally damaged by absorbed energy, while the lighter surrounding tissue may be spared. However, such techniques may use specific combinations of laser and target characteristics.

Certain types of lasers may be effective in producing desirable effects when absorbed by certain tissue structures. For example, KTP lasers and pulsed dye lasers can be effectively absorbed by vascular lesions to produce cosmetic improvements. The electromagnetic radiation produced by these lasers can target the vascular hemoglobin to generate local heating of the lesions. Vascular lesions include, e.g., port wine stains and hemangiomas. However, these structures are often located at some depth below the skin surface. The KTP lasers and the pulsed dye lasers, among others, are highly absorbed, and generally do not penetrate deeply into the skin, e.g., sometimes only 1 or 2 mm. Surface cooling can be important when using these lasers to avoid excessive damage to the epidermis, while providing sufficient power to allow the radiation to make a deeper penetration. This requirement can make the treatment more complex and less effective, and some vascular structures may be located too deep below the surface to be treated effectively with such lasers.

Therefore, there may be a need to provide an apparatus that is capable of directing the energy provided by highly-absorbed lasers to specific structures which may be located deep below the skin surface, while avoiding the effectuation of excessive damage to the epidermis. There may also be a need to avoid or reduce the above-described deficiencies.

### SUMMARY OF EXEMPLARY EMBODIMENTS OF THE INVENTION

The invention is defined by the claims.

It is one of the objects of the present invention to provide exemplary apparatus that can combine safe and effective treatment for an improvement of the dermatological disorders with minimum side effects. Another object of the present invention is to provide exemplary apparatus that generates a region of thermal damage deep below the skin surface that can be located accurately while causing only a small amount of damage to other nearby tissues.

These and other objects can be achieved using the exemplary embodiment of the apparatus according to the present invention, in which a first ablative radiation sources can be configured to provide a beam to generate a small hole that penetrates the epidermis and terminates near the target area that is to receive thermal damage. A second, non-ablative radiation beam can then be directed into the hole where it can easily pass through the ablated passageway, and be absorbed primarily in the vicinity of the bottom of the hole. This exemplary procedure can provide enhanced and highly localized thermal damage adjacent to the target area. The second radiation beam can be applied less than about three seconds, or preferably less than about one second, after the first radiation beam is applied to the skin tissue.

In a further exemplary embodiment of the present invention, the skin tussue surrounding the area to be treated can be cooled and/or frozen. This can provide an analgesic effect and reduce the extent of lateral thermal damage surrounding the ablated volume. If the tissue is frozen, it may be more mechanically rigid and improve the stability of the hole to provide a clearer passage for the second radiation beam.

In yet another exemplary embodiment of the present invention, a vacuum chamber may be used to stretch the skin surface over the target structure located beneath the skin surface. A first ablative radiation source may be configured to generate a small hole that penetrates the epidermis and terminates near the target area. A second, less-ablative radiation source can then direct a beam into the hole, where it can be absorbed primarily near the target area. The vacuum can then be released, allowing the stretched skin surface to relax and closing the hole more rapidly.

These and other objects, features and advantages of the present invention will become apparent upon reading the following detailed description of embodiments of the invention, when taken in conjunction with the included drawings.

### BRIEF DESCRIPTION OF THE DRAWINGS

For a more complete understanding of the present invention and its advantages, reference is now made to the following description, taken in conjunction with the accompanying drawings, in which:
FIG. 1 is a black diagram of an exemplary apparatus;
FIG. 2A is a cross-sectional view of an exemplary use of the apparatus of FIG. 1 and a method which can be performed by such apparatus to form an ablated hole terminating near a target structure;
FIG. 2B is a cross-sectional view of an exemplary ablated hole
   after the formation thereof in FIG. 2A;
FIG. 2C is a cross-sectional view of an exemplary use of the apparatus of FIG. 1 and the method which may be performed by such apparatus to form an exemplary thermal damage pattern;
FIG. 2D is a cross-sectional view of an exemplary thermal damage pattern that may be produced
   after the irradiation shown in FIG. 2C;
FIG. 3 is a cross-sectional view of another exemplary damage pattern that may be used to treat a larger vascular defect formed using the exemplary apparatus, and
FIG. 4 shows a cross-sectional view of the exemplary system and apparatus that may be used to promote more rapid closure of an ablated hole.

Throughout the drawings, the same reference numerals and characters, unless otherwise stated, are used to denote like features, elements, components, or portions of the illustrated embodiments. Moreover, while the present invention will now be described in detail with reference to the Figures, it is done so in connection with the illustrative embodiments.

### DETAILED DESCRIPTION OF THE PREFERRED EMBODIMENTS

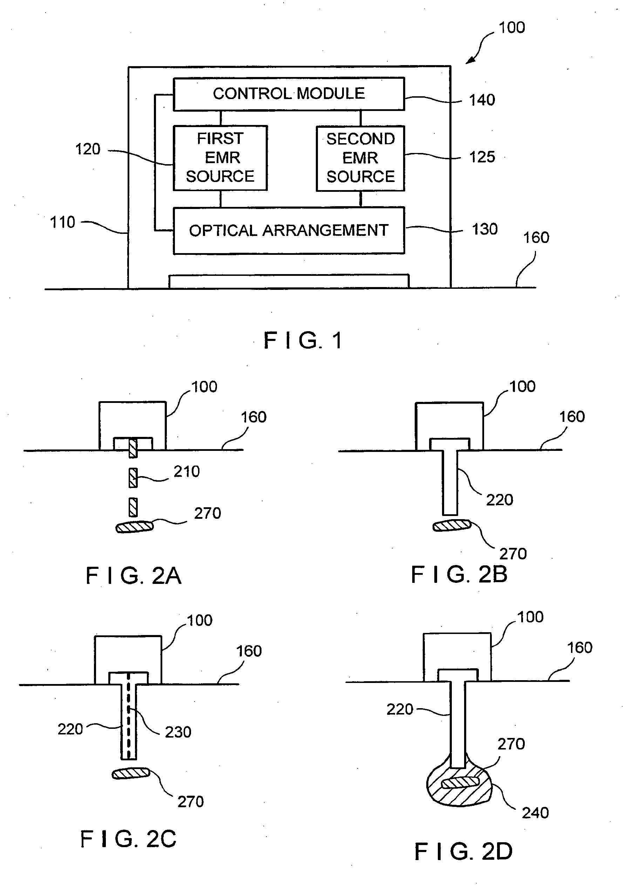

FIG. 1 shows an exemplary apparatus 100 that may be used to provide a dermatological treatment. The apparatus 100 can include a housing 110 that may be positioned in contact with the surface of the skin 160 over an area to be treated. The apparatus 100 also can include a first electromagnetic radiation ("EMR") source 120, a second EMR source 125, an optical arrangement 130, and a control module 140. These components may be situated within the housing 110 as illustrated in FIG. 1, or part or all of any of them may be located outside the housing 110. The control module 140 can be in communication with the EMR sources 120, 125, each of which in turn can be operatively connected to the optical arrangement 130. The control module 140 can also be in electrical and/or optical communication with the optical arrangement 130.

In one exemplary embodiment of the present invention, the control module 140 can be in wireless communication with the EMR sources 120 and/or 125. In another exemplary embodiment of the present invention, the control module 140 may be in wired communication with one or more of the EMR sources 120, 125. In yet another exemplary embodiment of the present invention, the control module 140 can be located outside of the housing 110. In still another exemplary embodiment of the present invention, the first EMR source 120 and/or the second EMR source 125 may be located outside of the housing 110. In a further exemplary embodiment of the present invention, the control module 140 and one or both of the EMR sources 120, 125 are each located outside of the housing 110.

The control module 140 can provide application-specific settings to the first EMR source 120 and the second EMR source 125. The first and second EMR sources 120, 125 can be configured to receive these settings, and generate a first EMR and a second EMR, respectively, based on these settings. The energy produced by the first and second EMR sources 120, 125 can be optical radiation, which can be focused, collimated and/or directed at least in part by the optical arrangement 130 towards the surface of the skin 160. Examples of the settings include, but are not limited to, the wavelength of the EMR, the energy delivered to the skin, the power delivered to the skin, the pulse duration for each EMR pulse, the fluence of the EMR delivered to the skin, the number of EMR pulses, the delay between individual EMR pulses, the beam profile of the EMR, etc.

The first EMR source 120 can be capable of generating the first EMR so as to ablate skin tissue. This first EMR source 120 can be, for example, a CO₂ laser or an Er:YAG laser. The second EMR source 125 can be capable of causing thermal damage to the skin tissue. The second EMR source 125 is the same laser as the first EMR source 120 operated at a lower peak power level or with one or more different parameters such that it will generate thermal damage when directed into skin tissue.

An exemplary method providing exemplary steps using the exemplary system and apparatus of the present invention are illustrated in FIGS. 2A-2D. In FIG. 2A, the apparatus 100 can be placed on the skin surface over the target 270. The target 270 can be, e.g., a vascular lesion such as a hemangioma, a tattoo or other pigmentation, or any other structure within the skin tissue that can be beneficially affected by local heating. A beam 210 from the first EMR source 120 can be directed into the skin toward the target 270. The characteristics of the beam 210 can be selected such that it ablates tissue that it contacts, which produces a narrow hole 220 as shown in FIG. 2B. The parameters of the beam 210 such as, for example, fluence or pulse duration, may be adjusted so that the bottom of the narrow hole 220 lies close to the target 270. In certain exemplary embodiments of the present invention, the bottom of the hole 270 may lie just above the target 270, and/or it may be located within the target 270. The diameter of the ablated hole 220 can be between, e.g., approximately 0.2 mm and 0.7 mm, or more preferably, between about 0.3mm and 0.5 mm. The depth of the hole can be selected based on the depth of the desired target to be thermally treated or damaged.

After the hole 220 is formed by the ablative beam 210 generated by the first EMR source 120, a nonablative beam 230 (provided, e.g., by the second EMR source 125) can be directed through the hole to the bottom as shown in FIG. 2C. Because the beam 230 is traveling primarily through an ablated hole, a small amount of the energy associated therewith it may be absorbed until the beam 230 reaches the bottom of the hole 220. At the bottom of the hole 220, the beam 230 will be absorbed, generating a zone of thermal damage 270. The duration of the applied pulse of the second EMR beam 230 can be longer than that of the ablative beam 210. This exemplary technique can provide a longer heating interaction at a lower energy level, which is beneficial for creating a significant amount of thermal damage.

If the second EMR source 125 generates radiation that is highly absorbed, then the zone of thermal damage 270 may be localized around the bottom of the hole 220. This exemplary method and apparatus can facilitate the thermal damage zones to be created at precisely predefined locations. In addition, the degree of a local damage can be significant because most of the energy generated in the second EMR source beam 230 can travel freely through the hole 220, and not be absorbed until it reaches the bottom.

The second EMR beam 230 can be directed into the hole 220 soon after the hole 220 is formed by the ablative beam 210. The time between the generation of the two beams 210, 230 should be as short as possible, because the hole 220 begins to collapse or close quickly, e.g., on the order of about three seconds or less, or preferably less than about one second.

Ablative holes produced using a CO₂ laser have been observed to close very quickly. A hole made using the CO₂ laser having a wavelength of 10.6 µm, an applied energy of about 0.4 J, a one-millisecond pulse, and a focal diameter of about 0.2 mm may produce an ablated zone approximately 0.3 mm in diameter that extended about 1.4-4 mm into the skin from the surface. A thermal damage zone about 0.05 to 0.1 mm can be observed around the ablated hole. The hole may be observed to heal rapidly, within about 1-2 days, with no scarring. These observations indicate that this exemplary technique can be performed without increasing the risk of scarring or infection significantly because of the fast healing response of the small holes.

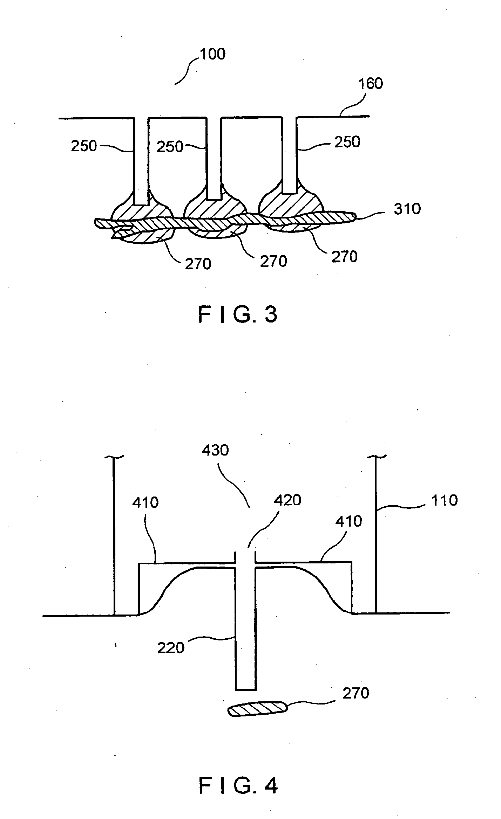

FIG. 3 illustrates an implementation of a further exemplary embodiment, in which a larger structure 310 such as, e.g., a port wine stain, may be thermally damaged with a high precision. This can be achieved by providing a plurality of ablated holes 250, each ending close to or at the structure 310. The second EMR beam 230, applied soon after formation of the holes 250, can create several adjacent areas of the thermal damage 270. These areas can contain or significantly affect the bulk of the structure 310. In this manner, large regions of thermal damage can be precisely generated well below the surface of the skin, with very little damage occurring to the tissue above the damaged region.

In a further exemplary embodiment a vacuum-based housing arrangement can be provided to more accurately align the EMR beams with the target site located on the surface of the skin. An exemplary configuration for this arrangement is illustrated in FIG. 4. For example, a recessed chamber 410 containing an orifice 420 can be formed at the lower portion of the housing 110. The housing 110 can be placed over the target structure 270, and a vacuum may be provided in the region 430 above the orifice 420. This vacuum can draw the skin surface 160 into the chamber 410 until the skin surface 160 contacts the upper surface of the chamber 410. The housing 110 can be positioned so that the target structure 270 is located directly beneath the orifice 420. In this manner, the vacuum is capable of holding the skin surface 160 firmly against the chamber 410 to maintain a more precise alignment of the first (ablative) and second (thermally damaging) EMR beams with the target structure 270.

An additional advantage of the exemplary embodiment of the present invention illustrated in FIG. 4 can be that the effective area of the skin surface 160 that is penetrated during generation of the ablated hole 220 can be reduced quickly. When the skin surface 160 is pulled up into the chamber 410 by a vacuum, it may be stretched. This stretching may persist while the first and second EMR beams 210, 230 penetrate into the skin tissue. After the vacuum is released, the skin surface 160 can relax and regain its original shape. This relaxation at the skin surface can cause the ablated hole 220 to shrink. This mechanical shrinkage can improve the posttreatment appearance of the skin quickly and lead to more rapid healing of the tissue around the ablated hole 220.

In a still further exemplary embodiment, a cooling arrangement can be provided to cool or freeze a portion of the skin to be treated before ablating a hole in the skin. Such cooling arrangement can provide cooling, e.g., using conventional contact or spray cooling techniques. Cooling the skin before ablation can provide an analgesic effect for the ablation procedure. If sufficient cooling is applied to at least partially freeze a portion of the dermal tissue, this can mechanically stabilize the tissue surrounding an ablated hole to allow more accurate alignment of the second EMR beam. It may also reduce the extent of lateral thermal damage produced by the ablation (e.g., damage along the sides of an ablated hole).

The foregoing merely illustrates the principles of the invention. Various modifications and alterations to the described embodiments will be apparent to those skilled in the art in view of the teachings herein.

## Claims

1. An apparatus for dermatological treatment, comprising:
a first arrangement configured to generate a first electromagnetic radiation and at least one second electromagnetic radiation, and
a second arrangement configured to:
direct the first electromagnetic radiation onto a particular location on a skin tissue so as to remove a volume thereof, and
direct the at least one second electromagnetic radiation onto the particular location after directing the first electromagnetic radiation thereon,
wherein the first radiation is provided as an ablative beam and wherein the at least one second electromagnetic radiation is provided as a nonablative beam; and
wherein the first arrangement is a single laser source.

2. The apparatus of claim 1, wherein the electromagnetic radiation is provided by one of a CO2 laser or an ER:YAG laser.

3. The apparatus of any preceding claim, wherein a first peak power level associated with the first electromagnetic radiation is greater than a second peak power level associated with the at least one second electromagnetic radiation.

4. The apparatus of any preceding claim, further comprising a cooling arrangement configured to cool at least a portion of the skin tissue containing the particular location.

5. The apparatus of claim 4, wherein the cooling arrangement is configured to freeze at least the portion of the tissue.

6. The apparatus of any of claims 1-5, further comprising a vacuum arrangement configured to pull a portion of skin tissue containing the particular location in the direction of the optical arrangement.

7. The apparatus of any preceding claim, wherein the second arrangement is configured to direct the at least one second electromagnetic radiation less than about three seconds after directing the first electromagnetic radiation.

8. The apparatus of any of claims 1-8, wherein the second arrangement is configured to direct the at least one second electromagnetic radiation less than about one second after directing the first electromagnetic radiation.

## Patentansprüche

1. Vorrichtung für dermatologische Behandlung, Folgendes umfassend:
eine erste Anordnung, konfiguriert, um eine erste elektromagnetische Strahlung und wenigstens eine zweite elektromagnetische Strahlung zu erzeugen und
eine zweite Anordnung, konfiguriert, um:
die erste elektromagnetische Strahlung auf eine bestimmte Stelle eines Hautgewebes auszustrahlen, um ein Volumen davon zu entfernen und
die wenigstens eine zweite elektromagnetische Strahlung auf die bestimmte Stelle auszustrahlen, nachdem die erste elektromagnetische Strahlung darauf ausgestrahlt worden ist,
wobei die erste Strahlung als ein ablativer Strahl bereitgestellt wird und wobei die wenigstens eine zweite elektromagnetische Strahlung als ein nichtablativer Strahl bereitgestellt wird; und
wobei die erste Anordnung eine einzelne Laserquelle ist.

2. Vorrichtung nach Anspruch 1, wobei die elektromagnetische Strahlung von einem CO₂-Laser oder einem Er:YAG-Laser bereitgestellt wird.

3. Vorrichtung nach einem der vorhergehenden Ansprüche, wobei eine erste Spitzenleistungsstufe, die mit der ersten elektromagnetischen Strahlung assoziiert ist, größer ist als eine zweite Spitzenleistungsstufe, die mit der wenigstens einen zweiten elektromagnetischen Strahlung assoziiert ist.

4. Vorrichtung nach einem der vorhergehenden Ansprüche, welche ferner eine Kühlungsanordnung umfasst, die konfiguriert ist, um wenigstens einen Teil des Hautgewebes, welches die bestimmte Stelle umfasst, zu kühlen.

5. Vorrichtung nach Anspruch 4, wobei die Kühlungsanordnung konfiguriert ist, um wenigstens den Teil des Gewebes einzufrieren.

6. Vorrichtung nach einem der Ansprüche 1-5, welche ferner eine Ansauganordnung umfasst, die konfiguriert ist, um einen Teil des Hautgewebes, welches die bestimmte Stelle umfasst, in Richtung der optischen Anordnung zu ziehen.

7. Vorrichtung nach einem der vorhergehenden Ansprüche, wobei die zweite Anordnung konfiguriert ist, um die wenigstens eine zweite elektromagnetische Strahlung weniger als etwa drei Sekunden nach dem Ausstrahlen der ersten elektromagnetischen Strahlung auszustrahlen.

8. Vorrichtung nach einem der Ansprüche 1-6, wobei die zweite Anordnung konfiguriert ist, um die wenigstens eine zweite elektromagnetische Strahlung weniger als etwa eine Sekunde nach dem Ausstrahlen der ersten elektromagnetischen Strahlung auszustrahlen.

## Revendications

1. Appareil de traitement dermatologique, comportant :
un premier agencement configuré pour générer un premier rayonnement électromagnétique et au moins un second rayonnement électromagnétique, et
un second agencement configuré pour :
diriger le premier rayonnement électromagnétique vers un emplacement particulier sur un tissu cutané de manière à retirer un volume de celui-ci, et
diriger le au moins un second rayonnement électromagnétique vers l'emplacement particulier après avoir dirigé le premier rayonnement électromagnétique sur celui-ci,
dans lequel le premier rayonnement est délivré sous la forme d'un faisceau ablatif et dans lequel le au moins un second rayonnement électromagnétique est délivré sous la forme d'un faisceau non ablatif, et
dans lequel le premier agencement est une source laser unique.

2. Appareil selon la revendication 1, dans lequel le rayonnement électromagnétique est délivré par au moins un élément parmi un laser CO₂ ou un laser Er:YAG.

3. Appareil selon l'une quelconque des revendications précédentes, dans lequel un premier niveau de puissance de crête associé au premier rayonnement électromagnétique est supérieur à un second niveau de puissance de crête associé au au moins un second rayonnement électromagnétique.

4. Appareil selon l'une quelconque des revendications précédentes, comportant en outre un agencement de refroidissement configuré pour refroidir au moins une portion du tissu cutané contenant l'emplacement particulier.

5. Appareil selon la revendication 4, dans lequel l'agencement de refroidissement est configuré pour congeler au moins la portion du tissu.

6. Appareil selon l'une quelconque des revendications 1 à 5, comportant en outre un agencement sous vide configuré pour tirer une portion de tissu cutané contenant l'emplacement particulier dans la direction de l'agencement optique.

7. Appareil selon l'une quelconque des revendications précédentes, dans lequel le second agencement est configuré pour diriger le au moins un second rayonnement électromagnétique moins d'environ trois secondes après avoir dirigé le premier rayonnement électromagnétique.

8. Appareil selon l'une quelconque des revendications 1 à 6, dans lequel le second agencement est configuré pour diriger le au moins un second rayonnement électromagnétique moins d'environ une seconde après avoir dirigé le premier rayonnement électromagnétique.
